(19) **Europäisches Patentamt**

**European Patent Office**

**Office européen des brevets**

(11) **EP 1 408 935 B1**

(12) **EUROPEAN PATENT SPECIFICATION**

(45) Date of publication and mention
of the grant of the patent:
**26.10.2005 Bulletin 2005/43**

(21) Application number: **01272036.3**

(22) Date of filing: **19.12.2001**

(51) Int Cl.⁷: $A61K\ 9/68$, $A23G\ 3/30$

(86) International application number:
**PCT/EP2001/015141**

(87) International publication number:
**WO 2002/051391 (04.07.2002 Gazette 2002/27)**

(54) **PROCESS FOR THE PREPARATION OF MEDICATED GUMS**

VERFAHREN ZUR HERSTELLUNG VON MEDIZINISCHEM KAUGUMMI

PROCEDE DE FABRICATION DE GOMMES MEDICAMENTEUSES

(84) Designated Contracting States:
**AT BE CH CY DE DK ES FI FR GB GR IE IT LI LU MC NL PT SE TR**

(30) Priority: **22.12.2000 IT MI20002809**

(43) Date of publication of application:
**21.04.2004 Bulletin 2004/17**

(73) Proprietor: **PIERRE FABRE MEDICAMENT**
**92654 Boulogne Cédex (FR)**

(72) Inventor: **BADETTI, Rolando**
**I-20052 Monza (IT)**

(74) Representative: **Ahner, Francis et al**
**Cabinet Régimbeau**
**20, rue de Chazelles**
**75847 Paris cedex 17 (FR)**

(56) References cited:
**EP-A- 0 288 909       WO-A-01/19206**
**WO-A-96/03111       WO-A-98/19558**
**DE-A- 19 751 330       US-A- 4 000 321**

**Description**

The present invention concerns a process for the preparation of medicated gums, particularly a process for the preparation of base gum suitable to the use as medicated gum.

The base gum of the chewing gums is a mixture generally comprising elastomers, resins, plasticizers, insoluble audjuvants, food antioxidants. All the base gums addressed to the preparation of chewing gums clearly must be consistent with rules for the direct preparations for food use, and specifically in the case of the medicated chewing gums, the problems generated by the presence of active principle must be taken into consideration.

In order to prepare the chewing gums there were two different approaches. The first one consisted of warming the base gum with the above indicated composition between 45 and 50°C in order to soften it and to put it in a kneading machine, heated by the circulation of jacket hot water or steam. Other components were then added in order to obtain the finished chewing gum. After the rest the mass was hence further heated and then subjected to treatments for the preparation of pieces of chewing gum of the desired shape. This process has been resulted inadequate for the preparation of medicated chewing gums, because the use of heat, which is inadvisable for thermolabile active principles, was requested and furthermore it allowed an amount of active principle not consistent with the therapeutical dose to be included. As a matter of fact the amount to be included was limited to 15/20% of the total mass.

In the attempt of solving the above indicated problems, in the case of medicated chewing gums both for pharmaceutical use and for nutritional use, a second process for the preparation of chewing gums which consists of a technique of powder compression was envisaged. The base gum was powdered and then mixed with one or more active principles, sweeteners, fragrances and various other components in order to obtain a homogenous mixture. The obtained mixture was then passed to a tabletting machine, in order to be transformed into tablets. In such a way the heat is not used and the thermolabile active principles are preserved, but these processes presently used cannot allow an active principle amount more than 20% to be included. As a matter of fact there are active principles, which have to be in amount above 20%, and in some cases up to 30% to be effective. When a greater amount of active principle is tried to be included the percentage of the gum base has to be increased, thereby the finished medicated gum has such a size to be unsuitable for the prefixed use. Some powdered base gums, which are commercially available, contain only 50% of base gum, the remainder being excipients as sorbitol magnesium stearate, calcium carbonate, etc and such gums having a low percentage of pure gum allow a limited amount of active principle to be included.

International Publication WO 98/19558 discloses the cryogenic milling of gum base at temperatures which reach minus 20, minus 50 or minus 100°C using liquid nitrogen. The gum base is ground in a jet mill in nitrogen gas at minus 70°C and recovered particles averaged 50 microns in size. The ground gum is loaded with up to 40% of liquid active principle and tablets may be formed.

It was surprisingly found out that through a particular treatment of making in powder form of the base gum, which has to be subjected to the compression together with other ingredients for the preparation of medicated gums, a finished medicated gum is obtained which overcomes the drawback of maximum amount of the active principle to be included in the gum.

The object of the present invention is therefore to provide a process which allows a base gum of high concentration to be obtained in such a way to include in the final chewing gum up to 30/35% of active principle and that the obtained gum has a size able to satisfy the compliance of the user.

The above indicated object is attained through a process as indicated in claim 1. Further advantages of the invention are achieved through the characteristics indicated in the dependent claims.

The process according to the present invention comprises the following steps:

   a) making in powder form the base gum in a grinding chamber;
   b) mixing the obtained gum with one or more active principles and additives, in order to have a homogenous mixture,
   c) compressing the obtained mixture in tablets of desired sizes,

characterized in that the base gum before being made in powder form is frozen at a temperature between -70 to -50°C with a freezing liquid positioned between the grinding chamber and the loading chamber, said freezing liquid being continuously sprayed during the grinding step to stop the heat produced by friction, in order to keep the product temperature during the grinding and at the output between -70 and -50°C.

The term "base gum" is intended as meaning an initial product of waxy appearance, said product being solid at room temperature and being softer and softer as the temperature increases, comprising elastomers, resins, plasticizers, insoluble audjuvants and food antioxidants.

Advantageously the loading chamber according to the present invention is a mill hopper and the milling chamber is a hammer mill. Preferably the freezing liquid according to the invention is liquid nitrogen the temperature of which is -170°C. The ground base gum obtained by the process according to the present invention is in a concentration of gum comprised between 95 and 100%. It is therefore evident that the powdered gum according to the present invention is in such a gum concentration that this concentration allows it to include the active principles in amount above 20%. After the grinding step the powder has a granulometry which is dependent on the screen mesh used in the grinding chamber and it is preferably below 3 mm, and more preferably at least 50% of the obtained particles are below 1mm. Advantageously during the grinding step the freezing liquid preferably liquid nitrogen, is continuously sprayed in order to prevent the grinding machine of the grinding chamber from being heated by friction. The regulation of the temperature of the ground product is carried out through a temperature sensing device suitable to adjust opening/closing of the freezing liquid inlet valve. Preferably the nitrogen flow is adjusted by a solenoid valve driven by a thermostatic probe positioned at the outlet of the grinding machine.

According to the present invention the ground product has to be at a temperature between -70°C and -50°C. If the temperature increases above -50°C the ground gum is coarser, while if the temperature decreases below -70°C the ground gum becomes finer. Therefore when the temperature increases above -50°C, the probe opens the valve which allows the freezing liquid to be passed in said freezing liquid decreasing the temperature of the product and when the temperature decreases below -70°C the probe closes the freezing liquid inlet valve.

The base gum powder is then unloaded from the mill in a polyethylene bag placed in a suitable container, preferably of pressed cardboard. The amount for each container corresponds to about 20 kg. Due to the fact that the ground gum comes out from the grinding chamber at a temperature between -50°C and -70°C the container has to be closed and the gum is let reach the room temperature.

Preferably the active principles mixed with the gum are selected from the group consisting of ascorbic acid, sodium ascorbate, acetyl salicilic acid, ibuprofen, paracetamol, Dextrometorfan bromide, dimenhydrinate, nicotine, thyrotricine, acetylcysteine, ginger, ephedrine, D-pseudo ephedrine, valerian, ranitidine, cethirizine, loratidine, chlorexidine, tibenzonium iodide, benzamidine, sucralfate, cetylpiridinium, hydropropizine, sodium flouride, benzetonium chloride, green tea.

Preferably the additives mixed with the gum are selected from the group consisting of sweeteners, flavours, lubricants, antiadherents, fillers, etc.

The mixing step b) is preferably carried out in a rotary powder mixer, selected between biconic mixer, "V" mixer or cubic mixer.

The so obtained mixture is preferably pressed with a rotary tabletting machine which transforms said mixture into tablets of sizes comprised of 8-28 mm.

Some illustrative embodiments now follow

## Example A

Preparation of base gum

Base gum, sold by GUM BASE S.p.A. of Lainate (Milano, Italy), is introduced in a loading hopper of a hammer mill, from which it is then passed to the grinding chamber. The grinding machine of the hammer mill is maintained at the temperature of -60°C through liquid nitrogen circulation, whose temperature is of -190°C. The grinding step is therefore carried out and at the output a base gum with a gum amount of 100% is obtained and the granule size for all the granulate is below 3 mm and the size of 50% of the granulate is below 1 mm. The gum is then unloaded and let reach the room temperature.

Example n. 1

In a "biconical" mixer the following powders are introduced:

| Base gum ground and obtained in Example A | mg 1050 | Gum |
|---|---|---|
| Ascorbic acid | mg 206 | Active Principle |
| Sodium ascorbate | mg 331 | Active Principle |
| Aspartame | mg 7 | Sweetener |
| Potassium Acesulphame | mg 5 | Sweetener |
| Sorbitol | mg 150 | Sweetener |
| Isomalt | mg 41 | Sweetener |
| Silica Precipitate | mg 40 | antiadherent |
| Talc | mg 40 | antiadherent |
| Magnesium Stearate | mg 30 | Lubricant |
| Powdered Orange flavour | mg 60 | Flavourant |

(continued)

| Powdered Tangerine Flavour | mg 40 | Flavourant |
|---|---|---|
| | mg 2000 | |

All the above ingredients are mixed for 20 minutes and the obtained powder is pressed with a tabletting machine obtaining gums of 2 g. In such a case the amount of the active principle (mg 537) is 26.85 % of the total amount.

Example n.2

In a "biconical" mixer the following powders are introduced:

| Base gum ground and obtained in Example A | mg 1065 | Gum |
|---|---|---|
| Coated Salicic acid | mg 516 | Active Principle |
| Aspartame | mg 6 | Sweetener |
| Potassium Acesulphame | mg 4 | Sweetener |
| Betacyclodextrin | mg 74 | Sweetener |
| Silica Precipitate | mg 40 | antiadherent |
| Talc | mg 40 | antiadherent |
| Powdered Spearmint Flavour | mg 50 | Flavourant |
| Ammonium glycirrinizate | mg 5 | Flavourant |
| | mg 1800 | |

All the above ingredients are mixed for 20 minutes and the obtained powder is pressed with a tabletting machine obtaining gums of 1.8 g. In such a case the amount of the active principle (mg 516) is 28.7 % of the total amount

Example n.3

In a "biconical" mixer the following powders are introduced:

| Base gum ground and obtained in Example A | mg 950 | Gum |
|---|---|---|
| Partially Hydrolized guar gum | mg 642 | Active Principle |
| Ascorbic acid | mg 15 | Active Principle |
| Sorbitol | mg 150 | Sweetener |
| Mannitol | mg 87 | Sweetener |
| Aspartame | mg 6 | Sweetener |
| Silica Precipitate | mg 20 | antiadherent |
| Talc | mg 30 | antiadherent |
| Magnesium Stearate | mg 35 | Lubricant |
| Powdered Apple flavour | mg 100 | Flavourant |
| Anhydrous Citric acid | mg 15 | Flavourant |
| | mg 2050 | |

All the above ingredients are mixed for 20 minutes and the obtained powder is pressed with a tabletting machine obtaining gums of 2.05 g. In such a case the amount of the active principle (mg 657) is 32.05 % of the total amount.

Example n.4

In a "biconical" mixer the following powders are introduced:

| Base gum ground and obtained in Example A | mg 850 | Gum |
|---|---|---|
| Granulate acacia fiber | mg 510 | Active Principle |
| Ascorbic acid | mg 30 | Active Principle |

(continued)

| | | |
|---|---|---|
| Garcina Cambogia dry extract | mg 26 | Active Principle |
| Glucomannose | mg 34 | Active principle |
| Chromium Picolinate | mg 0,5 | Active principle |
| Isomalt | mg 30 | Sweetener |
| Aspartame | mg 3 | Sweetener |
| Potassium Acesulphame salt | mg 2 | Sweetener |
| Silica Precipitate | mg 35 | antiadherent |
| Talc | mg 35 | antiadherent |
| Magnesium Stearate | mg 30 | Lubricant |
| E110 Aluminium lacquer | mg 4,5 | Dye |
| Powdered Orange flavour | mg 100 | Flavourant |
| anhydrous Citric acid | mg 10 | Flavourant |
| | mg 1700 | |

All the above ingredients are mixed for 20 minutes and the obtained powder is pressed with a tabletting machine obtaining gums of 1.7 g. In such a case the amount of the active principle (mg 600,5) is 35.32 % of the total amount.

Example n.5

In a "biconical" mixer the following powders are introduced:

| | | |
|---|---|---|
| Base gum ground and obtained in Example A | mg 645 | Gum |
| Magnesium Lactate | mg 112 | Active Principle |
| Potassium Gluconate | mg 212 | Active Principle |
| Xilitol | mg 60 | Sweetener |
| Aspartame | mg 2 | Sweetener |
| Potassium Acesulphame salt | mg 2 | Sweetener |
| Silica Precipitate | mg 30 | antiadherent |
| Talc | mg 30 | antiadherent |
| Magnesium Stearate | mg 25 | Lubricant |
| Powdered Lemon flavour | mg 15 | Flavourant |
| Powdered grapefruit flavour | mg 5 | Flavourant |
| Ammonium glycirrinizate | mg 2 | Flavourant |
| anhydrous Citric acid | mg 10 | Flavourant |
| | mg 1150 | |

All the above ingredients are mixed for 20 minutes and the obtained powder is pressed with a tabletting machine obtaining gums of 1.15 g. In such a case the amount of the active principle (mg 324) is 28.17 % of the total amount.

**Claims**

1. A process for the preparation of medicated gums comprising the steps of:

a) making in powder form the base gum in a grinding chamber;
b) mixing the obtained gum with one or more active principles and additives, in order to have a homogenous mixture,
c) compressing the obtained mixture in tablets of desired sizes,

**characterized in that** the base gum before being made in powder form is frozen at a temperature between -70 to -50°C with a freezing liquid positioned between the grinding chamber and the loading chamber, said freezing liquid being continuously sprayed during the grinding step to stop the heat produced by friction, in order to keep the product temperature during the grinding and at the output between -70 and -50°C.

2. The process according to claim 1, wherein the ground base gum obtained by the step a) has a concentration of gum comprised of 95-100%.

3. The process according to claim 1 or 2, wherein the freezing liquid is liquid nitrogen.

4. The process according to anyone of the preceding claims, wherein the loading chamber according to invention is a mill hopper and the grinding chamber is a hammer mill.

5. The process according to anyone of the preceding claims, wherein the powder entering into step b) has a granulometry below 3 mm, more preferably wherein at least 50% of obtained particles is below 1 mm.

6. The process according to anyone of the preceding claims, wherein the regulation of the temperature of the ground product is carried out through a temperature sensing device able to regulate opening/closure of inlet valve of the freezing liquid.

7. The process according to claim 6, wherein the flow of the freezing liquid is regulated by a solenoid valve guided by a thermostatic probe placed at the outlet of the grinding chamber.

8. The process according to anyone of the preceding claims, wherein the active principles mixed with the gum are selected from the group consisting of ascorbic acid, sodium ascorbate, acetyl salycilic acid, ibuprofen, paracetamol, Dextrometorfan bromide, dimenhydrinate, nicotine, thyrotricine, acetylcysteine, ginger, ephedrine, D-pseudoephedrine, valerian, ranitidine, cethirizine, loratidine, chlorexidine, tibenzonium iodide, benzamidine, sucralfate, cetylpiridinium, hydropropizine, sodium fluoride, benzetonium chloride, green tea.

9. The process according to anyone of the preceding claims, wherein the additives mixed with the gum are selected from the group consisting of sweeteners, flavours, lubricants, anti-adherents, fillers.

10. The process according to anyone of the preceding claims, wherein the mixing step b) is carried out in a rotary powder mixer, preferably selected between biconic mixer, "V" mixer or cubic mixer and the pressuring step c) is carried out in a rotary tabletting machine.

**Patentansprüche**

1. Verfahren zur Herstellung von mit Arzneimitteln versehenen Gummis, welches die Schritte umfasst:

   a) den Grundlagengummi in einer Mahlkammer in Pulverform zu überführen;
   b) den erhaltenen Gummi mit einem oder mehreren Wirkstoffen und Zusatzstoffen zu mischen, um eine homogene Mischung zu haben;
   c) die erhaltene Mischung zu Tabletten von gewünschten Größen zu verdichten,

   **dadurch gekennzeichnet, dass** der Grundlagengummi vor der Überführung in Pulverform bei einer Temperatur zwischen -70 und -50°C eingefroren wird mit einer Gefrierflüssigkeit, die zwischen der Mahlkammer und der Beladungskammer positioniert ist, wobei die Gefrierflüssigkeit während des Mahlschritts kontinuierlich versprüht wird, um die durch Reibung erzeugte Wärme zu stoppen, um die produkttemperatur während des Mahlens und beim Ausstoß zwischen -70 und -50°C zu halten.

2. Verfahren nach Anspruch 1, wobei der gemahlene Grundlagengummi, der durch den Schritt a) erhalten wird, eine Konzentration an Gummi von 95 bis 100% aufweist.

3. Verfahren nach Anspruch 1 oder 2, wobei die Gefrierflüssigkeit flüssiger Stickstoff ist.

4. Verfahren nach einem der vorangegangenen Ansprüche, wobei die Beladungskammer gemäß der Erfindung ein Mühlentrichter ist und die Mahlkammer eine Hammermühle ist.

**5.** Verfahren nach einem der vorangegangenen Ansprüche, wobei das in Schritt b) eintretende Pulver eine Korngrößenverteilung unter 3 mm aufweist, wobei mehr bevorzugt wenigstens 50% der erhaltenen Teilchen unter 1 mm liegt.

**6.** Verfahren nach einem der vorangegangenen Ansprüche, wobei die Regulierung der Temperatur des gemahlenen Produkts durch einen Temperaturmessfühler ausgeführt wird, welcher in der Lage ist, das Öffnen/Schließen eines Einlassventils der Gefrierflüssigkeit zu regulieren.

**7.** Verfahren nach Anspruch 6, wobei der Fluss der Gefrierflüssigkeit reguliert wird durch ein Magnetventil, welches durch einen Wärmewächter, der am Auslass der Mahlkammer platziert ist, betätigt wird.

**8.** Verfahren nach einem der vorangegangenen Ansprüche, wobei die mit dem Gummi gemischten Wirkstoffe aus der aus Ascorbinsäure, Natriumascorbat, Acetylsalicylsäure, Ibuprofen, Paracetamol, Dextrometorfanbromid, Dimenhydrinat, Nicotin, Thyrotricin, Acetylcystein, Ingwer, Ephedrin, D-Pseudoephedrin, Valerian, Ranitidin, Cethirizin, Loratidin, Chlorexidin, Tibenzoniumiodid, Benzamidin, Sucralfat, Cetylpiridinium, Hydropropizin, Natriumfluorid, Benzetoniumchlorid, grünem Tee bestehenden Gruppe ausgewählt werden.

**9.** Verfahren nach einem der vorangegangenen Ansprüche, wobei die mit dem Gummi gemischten Zusatzstoffe aus der aus Süßstoffen, Aroma- oder Geschmackstoffen, Gleitmitteln, Antihaftmitteln, Füllstoffen bestehenden Gruppe ausgewählt werden.

**10.** Verfahren nach einem der vorangegangenen Ansprüche, wobei der Mischschritt b) in einem Rotationspulvermischer, welcher vorzugsweise aus einem Doppelkonusmischer, "V"-Mischer oder Würfelmischer ausgewählt wird, ausgeführt wird und der Verdichtungsschritt c) in einer Rotationstablettiermaschine ausgeführt wird.

**Revendications**

**1.** Procédé de préparation de gommes médicamenteuses comprenant les étapes consistant à :

a) mettre sous forme de poudre la gomme de base dans une cuve de broyage ;
b) mélanger la gomme obtenue avec un ou plusieurs principes actifs et additifs, afin d'avoir un mélange homogène,
c) comprimer le mélange obtenu sous forme de comprimés de tailles souhaitées,

**caractérisé en ce que** la gomme de base, avant d'être mise sous forme de poudre, est congelée à une température comprise entre -70° et -50°C avec un liquide de congélation placé entre la cuve de broyage et la cuve de chargement, ledit liquide de congélation étant projeté en continu durant l'étape de broyage pour dissiper la chaleur produite par frottement, afin de maintenir la température du produit durant le broyage et à la sortie entre -70° et -50°C.

**2.** Procédé selon la revendication 1, dans lequel la gomme de base broyée obtenue dans l'étape a) a une concentration de gomme comprise entre 95-100%.

**3.** Procédé selon la revendication 1 ou 2, dans lequel le liquide de congélation est de l'azote liquide.

**4.** Procédé selon l'une quelconque des revendications précédentes, dans lequel la cuve de chargement selon l'invention est une trémie de broyeur et la cuve de broyage est un broyeur à marteaux.

**5.** Procédé selon l'une quelconque des revendications précédentes, dans lequel la poudre pénétrant dans l'étape b) a une granulométrie inférieure à 3 mm, mieux encore selon laquelle au moins 50% des particules obtenues font moins de 1 mm.

**6.** Procédé selon l'une quelconque des revendications précédentes, dans lequel la régulation de la température du produit broyé est réalisée au moyen d'un capteur de température capable de réguler l'ouverture/la fermeture de la vanne d'entrée du liquide de congélation.

**7.** Procédé selon la revendication 6, dans lequel le débit du liquide de congélation est régulé par une électrovanne commandée par une sonde thermostatique placée à la sortie de la cuve de broyage.

**8.** Procédé selon l'une quelconque des revendications précédentes, dans lequel les principes actifs mélangés avec la gomme sont choisis dans le groupe constitué par l'acide ascorbique, l'ascorbate de sodium, l'acide acétylsalicylique, l'ibuprofène, le paracétamol, le bromure de Dextrométorfane, le dimenhydrinate, la nicotine, la thyrotricine, l'acétylcystéine, le gingembre, l'éphédrine, la D-pseudoéphédrine, la valériane, la ranitidine, la céthirizine, la loratidine, la chlorhexidine, l'iodure de tibenzonium, la benzamidine, le sucralfate, le cétylpiridinium, l'hydropropizine, le fluorure de sodium, le chlorure de benzéthonium, le thé vert.

**9.** Procédé selon l'une quelconque des revendications précédentes, dans lequel les additifs mélangés avec la gomme sont choisis dans le groupe constitué par les édulcorants, les arômes, les lubrifiants, les anti-adhérents, les charges.

**10.** Procédé selon l'une quelconque des revendications précédentes, dans lequel l'étape de mélange b) est réalisée dans un mélangeur à poudres rotatif, de préférence choisi parmi un mélangeur biconique, un mélangeur en "V" ou un mélangeur cubique, et l'étape de pressurisation c) est réalisée dans une machine à comprimés rotative.